# EUROPÄISCHE PATENTSCHRIFT

(11) **EP 0 400 435 B1**
(45) Veröffentlichungstag und Bekanntmachung des Hinweises auf die Patenterteilung: **13.04.1994**
(21) Anmeldenummer: 90109560.4
(22) Anmeldetag: 19.05.1990
(51) Int. Cl.: C07C 219/04, C07C 219/22, C07D 295/08

(54) **Betainartige Kohlensäurehalbester**
Carbonic half-ester of betaine structure
Semi-ester-carbonate de structure bétaine

(30) Priorität: 01.06.1989 DE 3917830
(43) Veröffentlichungstag der Anmeldung: 05.12.1990
(73) Patentinhaber: BASF Aktiengesellschaft, 67063 Ludwigshafen (DE)
(72) Erfinder: Merger, Franz, Dr., D-6710 Frankenthal (DE); Frank, Juergen, Dr., D-6830 Schwetzingen (DE)

(56) Entgegenhaltungen:
- EP-A- 0 284 132
- GB-A- 751 129
- US-A- 2 635 100
- US-A- 3 708 539

## Beschreibung

Die vorliegende Erfindung betrifft betainartige Kohlensäurehalbester der allgemeinen Formel I 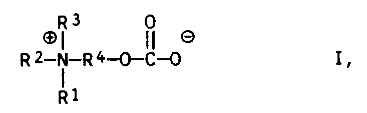
in der
R¹ und R² gleich oder verschieden sind und für eine C₁- bis C₂₅-Alkylgruppe, eine C₅- bis C₆-Cycloalkylgruppe, eine C₇- bis C₁₀-Aralkylgruppe und/oder die Phenyl- oder Naphthylgruppe stehen oder in der R¹ und R² miteinander verbunden sind und zusammen mit dem Stickstoffatom einen 5- bis 6-gliedrigen Ring bilden, der auch noch ein zusätzliches Stickstoff- oder Sauerstoffatom enthalten kann,
R³ eine C₁- bis C₁₀-Alkylgruppe ist und in der
R⁴ für eine C₁- bis C₂₅-Alkylengruppe, eine C₇- bis C₁₀-Aralkylengruppe, eine Phenylengruppe oder Oligo(oxyethylen)gruppe der allgemeinen Formel II 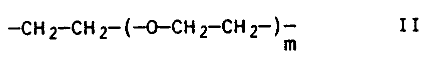
steht und in der
m eine ganze Zahl von 1 bis 20 bedeutet sowie ein Verfahren zu deren Herstellung.

Über amphotere Tenside wird in Ullmanns Encyklopädie der technischen Chemie, Band 22, Seite 497 bis 498, 4. Auflage, Verlag Chemie, Weinheim 1982 berichtet. Als Tenside im weiteren Sinne können auch Verbindungen betrachtet werden, welche aufgrund ihrer betainartigen, chemischen Struktur, beispielsweise als Haftvermittler in Klebstoffen dienen können. Da die oberflächenaktiven Eigenschaften der Tenside stark von deren chemischer Struktur abhängig sind, besteht ein Bedarf nach Tensiden mit neuer chemischer Struktur.

So bestand die Aufgabe, neue, betainartige Kohlensäurehalbester der allgemeinen Formel I sowie ein Verfahren zu ihrer Herstellung zur Verfügung zu stellen.

Dementsprechend wurden die eingangs definierten, betainartigen Kohlensäurehalbester der allgemeinen Formel I gefunden.

Des weiteren wurde ein Verfahren zur Herstellung der Kohlensäurehalbester der allgemeinen Formel I gefunden, daß dadurch gekennzeichnet ist, daß man ein Alkanolamin der allgemeinen Formel V 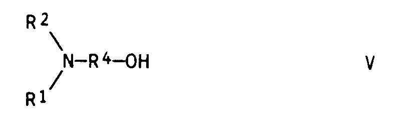
mit einem Kohlensäurediester der allgemeinen Formel VI 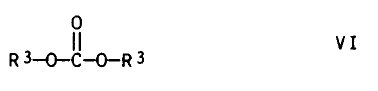
bei Temperaturen von 25 bis 200°C umsetzt.

In den erfindungsgemäßen betainartigen Kohlensäurehalbestern I, die allgemeine Formel stellt die zwitterionische Form dieser Verbindungen dar, können die Reste R¹ und R² gleich oder verschieden sein und für eine C₁- bis C₂₅-Alkylgruppe, eine C₅- bis C₆-Cycloalkylgruppe, eine C₇- bis C₁₀-Aralkylgruppe und/oder die Phenyl- oder Naphthylgruppe stehen.

R¹ und R² können auch miteinander verbunden sein und zusammen mit dem Stickstoffatom einen 5- bis 6-gliedrigen Ring bilden, der auch noch ein zusätzliches Stickstoff- oder Sauerstoffatom im Ring enthalten kann. Beispielsweise können R¹ oder R² mit dem Stickstoffatom einen Pyrrolidin-, Piperidin-, Piperazin-, Imidazolidin-, Oxazolidin- oder Morpholin-Ring bilden.

Als Reste R³ werden C₁- bis C₁₀-Alkylgruppen verwendet.

Bevorzugte Reste R³ sind C₁- bis C₄-Alkylgruppen, insbesondere n-Alkylgruppen und vornehmlich die Methylgruppe.

Der Rest R⁴ steht für eine C₁- bis C₂₅-Alkylengruppe. Weiterhin kann R⁴ für eine C₇- bis C₁₀-Aralkylen- oder eine Phenylengruppe stehen, sowie für Heteroalkylengruppen, wie die Oligo(oxyethylen)gruppe der allgemeinen Formel II 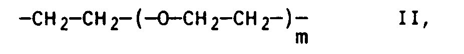
in der
m eine ganze Zahl von 1 bis 20 bedeutet, stehen.

Es versteht sich von selbst, daß bei der Definition der Reste R¹ bis R⁴ unter dem Begriff Alkyl- oder Alkylengruppe sowohl geradkettige als auch verzweigte Alkyl- oder Alkylengruppen verstanden werden.

Die erfindungsgemäßen betainartigen Kohlensäurehalbester der allgemeinen Formel I werden erfindungsgemäß durch Umsetzung der Alkanolamine der allgemeinen Formel V mit Kohlensäurediestern der allgemeinen Formel VI gemäß Gleichung (1) dargestellt: 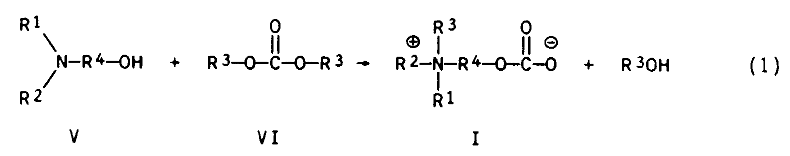
Beispielsweise können die folgenden Alkanolamine mit den Dialkylcarbonaten VI zu den erfindungsgemäßen Kohlensäurehalbestern I vorteilhaft umgesetzt werden:
N,N-Dimethylaminoethanol, N,N-Dimethylaminopropanol, N,N-Dimethylamino-n-butanol, N,N-Dimethylamino-n-octanol, N,N-Diethylaminoethanol, N,N-Diethylaminopropanol, N,N-Diethylamino-n-butanol, N,N-Diethylamino-n-pentanol, N,N-Diisopropylaminoethanol, N-n-Butyl-N-methylaminopropanol, N-Cyclohexyl-N-methylaminoethanol, N-Benzyl-N-n-propyl-aminoethanol, N-Hydroxyethylpyrrolidin, N-Hydroxyethylpiperidin, N-Hydroxyethylmorpholin, N-Hydroxypropylpyrrolidin, N-Hydroxypropylmorpholin, 3-Hydroxychinuclidin, N,N-Dimethyl-sec.-pentanol, N,N-Dimethylaminoneopentanol, 1-Hydroxymethyl-1-dimethylamino-cyclohexan, N,N-Dibutylaminoneopentanol, 3-Hydroxymethyl-3-dimethylaminomethyl-heptan, 3-Hydroxymethyl-3-(N-methyl-N-hexylamino)-heptan, N,N-Diphenylaminoethanol, N,N-Dibenzylaminopropanol, 4-(N,N-Dimethylamino)phenol, N,N-Dimethylamino-N-(4-methylphenyl)benzylalkohol, N,N-Dimethylamino-trioxiethylenethanol, N,N-Dipropylamin-tetraoxiethylen-ethanol, N,N-Dimethylaminohexadecanol, N,N-Dipropylamino-eicosanol.

Die Umsetzung wird im allgemeinen bei Temperaturen von 25 bis 200°C, vorzugsweise bei 50 bis 150°C und insbesondere bei 70 bis 130°C, durchgeführt. Die Umsetzung kann bei Atmosphärendruck oder unter erhöhtem Druck ausgeführt werden. Insbesondere bei Verwendung niedrigsiedender Kohlensäurediester VI, beispielsweise Dimethylcarbonat, oder niedrigsiedenden Lösungsmitteln, wie Tetrahydrofuran, kann es vorteilhaft sein, die Umsetzung unter dem Eigendruck des Reaktionsgemisches in einem Autoklaven auszuführen.

Als Lösungsmittel für die Umsetzung eignen sich insbesondere unter den Reaktionsbedingungen inerte, aprotische Lösungsmittel. Beispielsweise können Kohlenwasserstoffe wie Hexan, Heptan, Octan, Benzol, Toluol, Xylol oder Durol als auch Ether wie Tetrahydrofuran, Dioxan oder Dimethoxyethan und Ketone wie Aceton, Butanon, Pentanon, Methylisopropylketon, Ethylisobutylketon sowie Ester wie Ethylacetat, Ethylbutyrat oder Butylacetat verwendet werden. Halogenierte Kohlenwasserstoffe können ebenfalls als Lösungsmittel benutzt werden.

Vorzugsweise werden die Reaktanten jedoch in Abwesenheit eines Lösungsmittels miteinander umgesetzt. Dabei können sowohl das Alkanolamin V als auch der Kohlensäurediester VI in bis zu 25-fachem, vorzugsweise 10-fachem, molarem Überschuß bezüglich des betreffenden anderen Reaktanten eingesetzt werden. Unter diesen Bedingungen dient der jeweils überschüssige Reaktant gleichzeitig als Lösungsmittel. Bevorzugt wird jedoch mit einem Überschuß des Kohlensäurediesters VI bezüglich des Alkanolamins V gearbeitet. Es versteht sich von selbst, daß auch stöchiometrische Mengen der Reaktanten V und VI miteinander umgesetzt werden können, dies insbesondere dann, wenn die Reaktion in Anwesenheit eines Lösungsmittels durchgeführt wird.

Die Umsetzung kann vorteilhaft auch kontinuierlich betrieben werden, beispielsweise unter Anwendung eines Kaskaden- oder Rohrreaktors. Der Umsatz der Reaktanten unter den jeweiligen Reaktionsbedingungen kann dabei nach herkömmlichen analytischen Methoden, beispielsweise mittels Hochdruckflüssigchromatographie, bestimmt und danach die Verweilzeit festgelegt werden.

Wird die Umsetzung in Anwesenheit eines Lösungsmittels, beispielsweise Aceton, durchgeführt, so können die betreffenden betainartigen Kohlensäurehalbester I im allgemeinen kristallin erhalten und durch Filtration vom Lösungsmittel abgetrennt werden. Das Lösungsmittel kann, gegebenenfalls nach destillativer Aufarbeitung, wieder in den Prozeß zurückgeführt werden.

Wird einer der Reaktanten als Lösungsmittel verwendet und fällt dabei das Reaktionsprodukt nicht wie üblich aus, so kann dieses durch Verdünnen des Reaktionsaustrages mit einem inerten Lösungsmittel wie Aceton, Ethylacetat oder Hexan im allgemeinen praktisch vollständig ausgefällt und durch Filtration von der Mutterlauge abgetrennt werden. Diese kann destillativ aufgearbeitet und das darin enthaltene Lösungsmittel sowie der überschüssige Reaktant wieder verwendet werden.

Überraschenderweise liefert das erfindungsgemäße Verfahren die erfindungsgemäßen, betainartigen Kohlensäurehalbester I in guten Ausbeuten. Dies ist umso mehr erstaunlich als aus GB-PS 751 129 bekannt ist, daß Dimethylcarbonat mit tertiären Aminen unter Übertragung einer Methylgruppe zu Tetraalkylammonium-Carbonaten reagiert, d.h., es bildet sich das einbasige Salz der Monomethylkohlensäure, also Trialkylammonium-monomethylcarbonat. Außerdem ist bekannt, (vgl. Houben-Weyl, Methoden der organischen Chemie, Band VIII, Seite 109, Thieme, Stuttgart 1952), daß z.B. Dimethylcarbonat mit Alkoholen zu den entsprechenden symmetrischen oder asymmetrischen Dialkylcarbonaten umgeestert wird. Unter Berücksichtigung dieses Standes der Technik mußte erwartet werden, daß die Reaktion zwischen einem N,N-Dialkyl-hydroxyalkyl-amin und beispielsweise Dimethylcarbonat entweder
A) unter alleiniger Beteiligung der Aminofunktion zu N-Hydroxyalkyl-N,N-dialkyl-N-methylammonium-methylcarbonaten der Formel 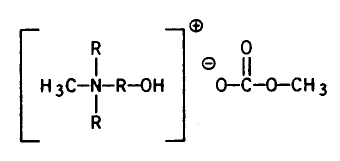 oder
B) unter alleiniger Beteiligung der Hydroxyfunktion unter Umesterung und Methanolabspaltung zu den Kohlensäurediestern der Formeln 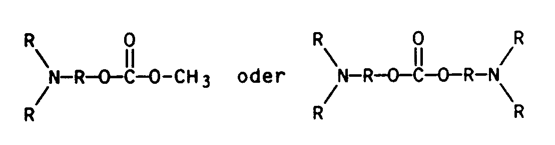 oder
C) unter Beteiligung beider funktioneller Gruppen zu den Verbindungen der Formel 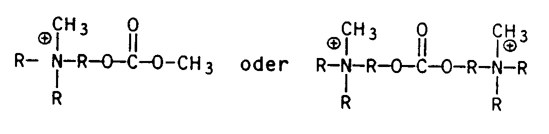 führen würde.
   Die zur Herstellung der betainartigen Kohlensäurehalbester benötigten Ausgangsmaterialien sind im Handel erhältlich oder können nach bekannten Verfahren hergestellt werden. Die Alkanolamine der Formel I können aus den entsprechenden Diaminen durch deren Umsetzung mit den entsprechenden Alkandiolen oder deren Derivaten, in denen die eine Hydroxyfunktion durch eine Schutzgruppe blockiert ist, in einer hydrierenden Aminierungsreaktion beispielsweise gemäß der Verfahren der EP-A 227 904 oder der US-A 3 708 539 hergestellt werden. Die Kohlensäurediester der Formel VI sind beispielsweise nach dem Verfahren der DE-C 2 334 736 erhältlich.
   Die erfindungsgemäßen betainartigen Kohlensäurehalbester I können als Tenside, insbesondere als Haftverbesserer in Klebstoffen, Verwendung finden.

### Beispiel 1

### N,N,N-Trimethyl-N-(hex-1-yl-6-monocarbonat)-ammonium

145 g (1 Mol) 6-Dimethylaminohexan-1-ol und 90 g (1 Mol) Dimethylcarbonat wurden 5 h bei 90°C gerührt. Nach Verdünnen mit wasserfreiem Aceton kristallisierte das Produkt III aus. Es wurde unter Stickstoff und unter Anwendung von vermindertem Druck abfiltriert und getrocknet.

Ausbeute: 42 %.

| Elementaranalyse: (C₁₀H₂₁NO₃) | | | | |
|---|---|---|---|---|
| ber: | C 59,1 | H 10,3 | N 6,9 | O 23,7 |
| gef: | C 58,5 | H 10,5 | N 7,0 | |

### Beispiel 2

### N,N,N-Trimethyl-N-(1-yl-ethan-2-monocarbonat)-ammonium

267 g (3 Mol) Dimethylaminoethanol und 270 g (3 Mol) Dimethylcarbonat wurden 72 h bei 70°C gerührt und anschließend mit wasserfreiem Aceton verdünnt. Dabei schied sich eine ölige Phase ab. Beim Verreiben des öls mit Aceton schied sich das Produkt IV kristallin ab. Es wurde wie beschrieben isoliert.

Ausbeute: 40 %.

| Elementaranalyse (C₆H₁₃NO₃) | | | | |
|---|---|---|---|---|
| ber: | C 49,0 | H 8,8 | N 9,5 | O 32,7 |
| gef: | C 48,4 | H 9,1 | N 9,4 | |

## Patentansprüche

1. Betainartige Kohlensäurehalbester der allgemeinen Formel I 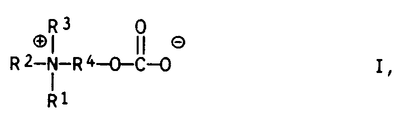 in der
R¹ und R² gleich oder verschieden sind und für eine C₁- bis C₂₅-Alkylgruppe, eine C₅- bis C₆-Cycloalkylgruppe, eine C₇- bis C₁₀-Aralkylgruppe und/oder die Phenyl- oder Naphthylgruppe stehen oder in der R¹ und R² miteinander verbunden sind und zusammen mit dem Stickstoffatom einen 5- bis 6-gliedrigen Ring bilden, der auch noch ein zusätzliches Stickstoff- oder Sauerstoffatom enthalten kann,
R³ eine C₁- bis C₁₀-Alkylgruppe ist,
und in der
R⁴ für eine C₁- bis C₂₅-Alkylengruppe, eine C₇- bis C₁₀-Aralkylengruppe, eine Phenylengruppe oder Oligo(oxyethylen)gruppe der allgemeinen Formel II 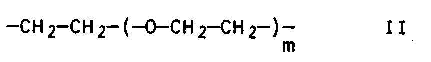 steht, in der
m eine ganze Zahl von 1 bis 20 bedeutet.

2. Betainartige Kohlensäurehalbester nach Anspruch 1, in denen R¹ und R² ausgewählt sind aus C₁- bis C₂₅-Alkylgruppen, R³ eine C₁- bis C₄-Alkylgruppe ist und in der R⁴ für eine C₁- bis C₂₅-Alkylengruppe steht.

3. Betainartige Kohlensäurehalbester nach Anspruch 1, in denen R¹ und R² ausgewählt sind aus C₁- bis C₂₅-Alkylgruppen, R³ eine Methylgruppe ist und in der R⁴ für eine C₁- bis C₂₅-Alkylengruppe steht.

4. N,N,N-Trimethyl-N-(hex-1-yl-6-monocarbonat)-ammonium der Formel III

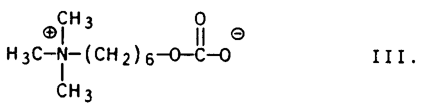

5. N,N,N-Trimethyl-N-(1-yl-ethan-2-monocarbonat)-ammonium der Formel IV

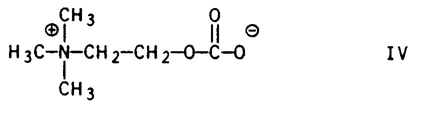

6. Verfahren zur Herstellung der Kohlensäurehalbester der allgemeinen Formel I, dadurch gekennzeichnet, daß man ein Alkanolamin der allgemeinen Formel V 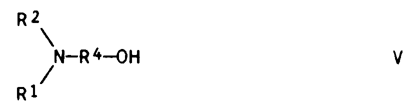 mit einem Kohlensäurediester der allgemeinen Formel VI 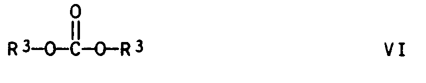 bei Temperaturen von 25 bis 200°C umsetzt.

7. Verfahren nach Anspruch 6, dadurch gekennzeichnet, daß man die Umsetzung bei 70 bis 130°C durchführt.

8. Verfahren nach Anspruch 6, dadurch gekennzeichnet, daß man als Kohlensäurediester Dimethylcarbonat verwendet.

## Claims

1. A betaine-like carbonic monoester of the Formula I 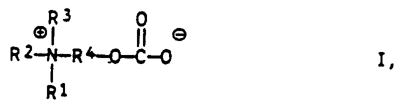 where
R¹ and R² are identical or different and are C₁- to C₂₅-alkyl, C₅- to C₆-cycloalkyl, C₇- to C₁₀-aralkyl and/or phenyl or naphthyl, or R¹ and R² are bonded to one another and, together with the nitrogen atom, form a 5- or 6-membered ring, which may also contain an additional nitrogen or oxygen atom,
R³ is C₁- to C₁₀-alkyl, and
R⁴ is C₁- to C₂₅-alkylene, C₇- to C₁₀-aralkylene, phenylene or oligo(oxyethylene) of the Formula II 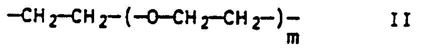 where
m is an integer from 1 to 20.

2. A betaine-like carbonic monoester as claimed in claim 1, where R¹ and R² are selected from C₁- to C₂₅-alkyl groups, R³ is C₁- to C₄-alkyl, and R⁴ is C₁- to C₂₅-alkylene.

3. A betaine-like carbonic monoester as claimed in claim 1, where R¹ and R² are selected from C₁- to C₂₅-alkyl groups, R³ is methyl, and R⁴ is C₁- to C₂₅-alkylene.

4. Trimethylammonio(1-hexyl-6-carbonate) of the formula III

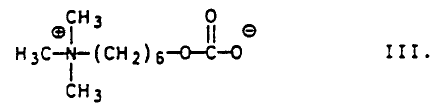

5. Trimethylammonio(1-ethyl-2-carbonate) of the formula IV

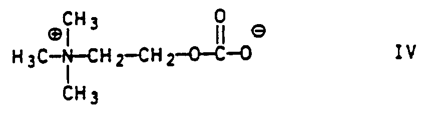

6. A process for the preparation of a carbonic monoester of the formula I, which comprises reacting an alkanolamine of the formula V 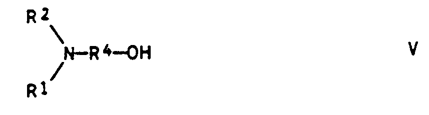 with a carbonic diester of the formula VI 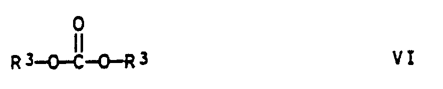 at from 25 to 200°C.

7. A process as claimed in claim 6, wherein the reaction is carried out at from 70 to 130°C.

8. A process as claimed in claim 6, wherein the carbonic diester used is dimethyl carbonate.

## Revendications

1. Semi-ester-carbonates de structure bétaïne de formule générale I 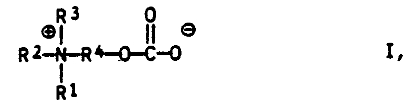 dans laquelle
R¹ et R² sont identiques ou différents et sont mis pour un groupe alkyle en C₁-C₂₅, un groupe cycloalkyle en C₅-C₆, un groupe aralkyle en C₇-C₁₀ et/ou les groupes phényle ou naphtyle, ou dans laquelle R¹ et R² sont liés l'un à l'autre et forment ensemble avec l'atome d'azote un cycle de 5 ou 6 atomes, qui peut également contenir encore un atome supplémentaire d'azote ou d'oxygène,
R³ est un groupe alkyle en C₁-C₁₀,
et dans laquelle
R⁴ est mis pour un groupe alkylène en C₁-C₂₅, un groupe aralkylène en C₇-C₁₀, un groupe phénylène ou oligo(oxyéthylène) de formule générale II 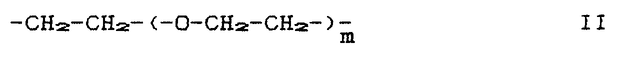 dans laquelle
m est un nombre entier compris entre 1 et 20.

2. Semi-ester-carbonates de structure bétaïne selon la revendication 1, dans lesquels R¹ et R² sont choisis parmi des groupes alkyle en C₁-C₂₅, R³ est un groupe alkyle en C₁-C₄, et R⁴ est mis pour un groupe alkylène en C₁ à C₂₅.

3. Semi-ester-carbonates de structure bétaïne selon la revendication 1, dans lesquels R¹ et R² sont choisis parmi des groupes alkyle en C₁-C₂₅, R³ est un groupe méthyle et R⁴ est mis pour un groupe alkylène en C₁-C₂₅.

4. N,N,N-triméthyl-N-(hex-1-yl-6-monocarbonate)-ammonium de formule III

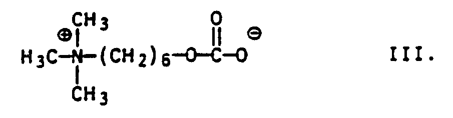

5. N,N,N-triméthyl-N-(1-yl-éthane-2-monocarbonate)-ammonium de formule IV

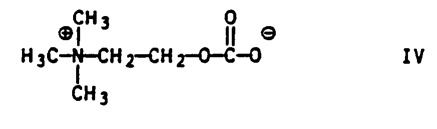

6. Procédé de préparation des semi-ester-carbonates de formule générale I, caractérisé en ce que l'on fait réagir à des températures comprises entre 25 et 200°C, une alcanolamine de formule générale V 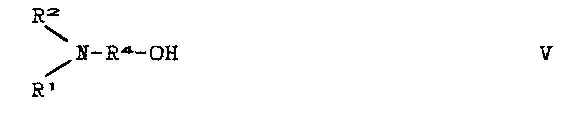 avec un diester-carbonate de formule générale VI

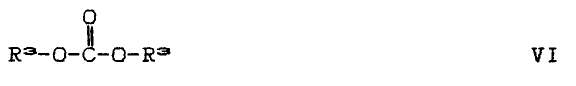

7. Procédé selon la revendication 6, caractérisé en ce que l'on effectue la réaction à des températures comprises entre 70 et 130°C.

8. Procédé selon la revendication 6, caractérisé en ce que l'on utilise comme diester-carbonate du carbonate de diméthyle.
